# EUROPEAN PATENT SPECIFICATION

(11) **EP 2 366 368 B1**
(45) Date of publication and mention of the grant of the patent: **06.04.2016**
(21) Application number: 09823534.4
(22) Date of filing: 26.10.2009
(51) Int. Cl.: A61F 13/20

(54) **ABSORBENT FOR TAMPON USE**
ABSORBENS ZUR TAMPONVERWENDUNG
CORPS ABSORBANT POUR TAMPON HYGIÉNIQUE

(30) Priority: 31.10.2008 JP 2008281592
(43) Date of publication of application: 21.09.2011
(73) Proprietor: Unicharm Corporation, Ehime 799-0111 (JP)
(72) Inventor: KIKUCHI, Akie, Kagawa 769-1602 (JP); YAMAKI, Koichi, Kagawa 769-1602 (JP); WATANABE, Hitoshi, Kagawa 769-1602 (JP)
(74) Representative: Dolleymores
(86) International application number: PCT/JP2009/068314
(87) International publication number: WO 2010/050424

(56) References cited:
- WO-A1-2004/049167
- JP-A- 2004 298 452
- JP-A- 2004 298 452
- JP-U- 54 024 993
- JP-U- 61 151 720
- US-A1- 2004 049 167

## Description

### [Technical Field]

The present invention relates to an absorber for a tampon, which is generated by compressing and molding a laminate member including a liquid permeable surface member and an absorptive member.

### [Background Art]

In an absorber for the tampon disclosed in Patent Document 1, an absorptive member before compressing and molding, the member constituting the absorber for the tampon, is structured so as to have a first portion at which a fiber density is low and a second portion at which a fiber density is high. Here, the first portion is provided at a side to be first inserted into a vaginal opening than the second portion.
An absorbent tampon is disclosed in Patent Document 2.

### [Prior Art Document]

### [Patent Document]

Patent Document 1: Japanese Unexamined Patent Application Publication No. 61-170462
Patent Document 2: PCT Patent Application No. WO 00/61052.

### [Summary of the Invention]

However, since such absorber for the tampon is structured so that the second portion is formed by jetting any liquid to the laminate member including the liquid permeable surface member and the absorptive member, equipment for jetting liquid to the laminate member mentioned above is required in order to manufacture such absorber for the tampon, and there has been a problem that running costs for generating the absorber for the tampon increase.

Therefore, the present invention has been made in view of the problem described above, and it is an object to provide an absorber for a tampon, which is capable of forming a region in which a fiber density is different from another one without jetting any liquid to a laminate member. The present invention provides the absorber for a tampon of independent Claim 1. The dependent claims specify preferred but optional features.

The first character of the present invention is summarized in that an absorber for a tampon, which is generated by compressing and molding a laminate member including a liquid permeable surface member and an absorptive member, the absorptive member before the compression and molding is comprised of: a first region which is a region at a side to be first inserted into a vaginal opening in a case where the absorber for tampon is divided into two sections in a longitudinal direction; and a second region which is a region at a side to be first removed from the vaginal opening in a case where the absorber for tampon is divided into two sections in a longitudinal direction; and an area of the first region is smaller than an area of the second region.

As has been described above, according to the present invention, there can be provided an absorber for the tampon, which is capable of forming a region in which a fiber density is different from another one without jetting any liquid to a laminate member.

### [Brief Description of Drawings]

[Fig. 1] Fig. 1 is a perspective view of an applicator for tampon in a state in which an absorber for a tampon, according to a first embodiment of the present invention, is inserted.
[Fig. 2] Fig. 2 is a perspective view of the absorber for the tampon, according to the first embodiment of the present invention.
[Fig. 3] Fig. 3 is a plan view of a laminate member before compressed and molded, of the absorber for the tampon, according to the first embodiment of the present invention.
[Fig. 4] Fig. 4 is a plan view of a laminate member before compressing and molding, of an absorber for the tampon, according to a second embodiment of the present invention.
[Fig. 5] Fig. 5 is a plan view of a laminate member before compressing and molding, of an absorber for the tampon, according to a third embodiment of the present invention.
[Fig. 6] Fig. 6 is a plan view of a laminate member before compressing and molding, of an absorber for the tampon, according to a fourth embodiment of the present invention.

### [Best Mode for Carrying out the Invention]

### (Absorber for Tampon, According to First Embodiment of the Present Invention)

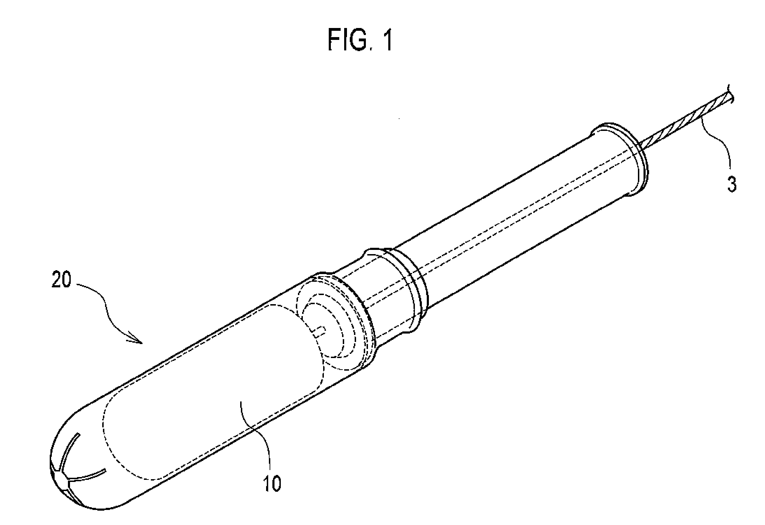

With referring to Fig. 1 to Fig. 3, an absorber 10 for a tampon, according to a first embodiment of the present invention, will be described.

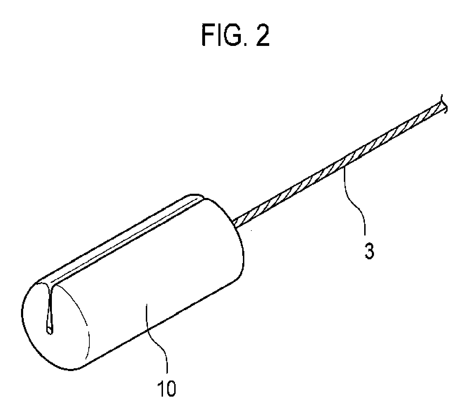

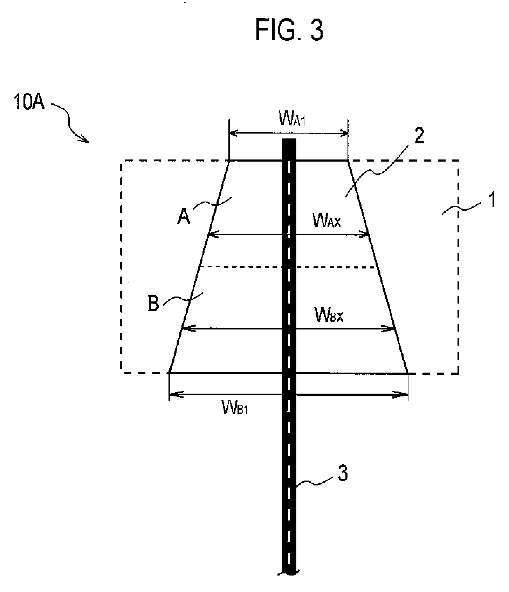

Fig. 1 is a perspective view of an applicator 20 for a tampon in a state in which an absorber 10 for the tampon, according to the illustrative embodiment, is inserted; Fig. 2 is a perspective view of the absorber 10 for the tampon after compressing and molding, according to the illustrative embodiment; and Fig. 3 is a plan view of a laminate member 10A before compressing and molding, of the absorber 10 for the tampon after compressing and molding, according to the illustrative embodiment.

As shown in Fig. 1, an absorber 10 for a tampon, according to the illustrative embodiment, is structured so as to be housed in an outer cylinder of an applicator 20 for a tampon.

In addition, the absorber 10 for the tampon, which is housed in the outer cylinder, is structured so as to be pushed from a rear side by means of a push-out member of the applicator 20 for the tampon, when the absorber is inserted into a vaginal opening, and then, is exposed to the outside of the outer cylinder by pushing and widening a piece part which is formed at an aperture of the applicator 20 for the tampon.

As shown in Fig. 2, a lead-out cord 3 extends from a rear end of the absorber 10 for the tampon, according to the illustrative embodiment, and as shown in Fig. 1, such lead-out cord 3 passes through the inside of a push-out member and then extends to the outside of the applicator 20 for the tampon.

Such absorber 10 for the tampon is structured so as to be generated by compression and molding a laminate member 10A shown in Fig. 3. For example, the absorber 10 for the tampon may be generated by compressing and molding the laminate member 10A (the absorptive member 2) shown in Fig. 3 after being rolled in a cylindrical manner.

Here, in a compression and molding process of the laminate member 10A, the laminate member 10A is compressed and molded in an approximately cylindrical shape by means of a molding die having a plurality of protrusive portions in a longitudinal direction, or alternatively, is compressed and molded in predetermined dimensions by being pressed in a respective one of a conveyance direction (MD direction) and a crossing direction (CD direction).

It is desirable that the dimension in a lengthwise direction of the absorber 10 for the tampon after compressing and molding is 30 mm to 60 mm. In a case where the dimension in the lengthwise direction of the absorber 10 for the tampon after compressing and molding is 30 mm or less, an area coming into contact with the vagina of the absorber 10 for the tampon is so small that menstrual blood cannot be absorbed sufficiently and leakage is likely to occur, or alternatively, in a case where the dimension in the lengthwise direction of the absorber 10 for the tampon after compressing and molding is 60 mm or more, the absorber 10 for the tampon inflates up to the vicinity of the virginal opening, thereby causing a wearer to feel discomfort.

As shown in Fig. 3, such laminate member 10A is comprised of a liquid permeable surface member 1, an absorptive member 2, and a lead-out cord 3 which is connected to the absorptive member 2 by means of a yarn.

The surface member 1 is employed as required, and may encompass rayon, cotton, two-component fiber, or alternatively, other known suitable natural fibers or synthetic fibers in the technical field.

Rayon, polyethylene, polypropylene or a mixture thereof is particularly suitable for use as such surface member 1. In addition, another surface member 1, in which one or more pores are provided on a surface, may be employed. It is preferable that a total weight of such surface member 1 is 8 g/m² to 40 g/m².

The absorptive member 2 may be comprised of: rayon (including conventional normal rayon and a different type of rayon); cotton; crushed wooden pulp; a chemically denatured/modified or cross-linked cellulose fiber; a synthetic resin; a tissue, or a liquid absorptive member which is generally used as an absorptive member such as peat moss, or a mixture thereof. Further, an ultra-absorptive material such as an ultra-absorptive polymer or an absorptive gel material may be incorporated in the absorptive member 2. It is preferable that a total weight of the absorptive member 2 is 100 g/m² to 1,200 g/m².

As shown in Fig. 3, the absorptive member 2 before compressing and molding is comprised of: a first region A which is a region at a side to be first inserted into a vaginal opening in a case where the absorber 10 for the tampon is equally divided into two sections in a longitudinal direction; and a second region B which is a region at a side to be first removed from the vaginal opening in the case where the absorber 10 for the tampon is equally divided into two section in the longitudinal direction.

In the absorptive member 2 before compressing and molding, an average dimension W_{A} in a widthwise direction in the first region A is shorter than an average dimension W_{B} in a widthwise direction in the second region B. That is, in the absorptive member 2 before compressing and molding, an area of the first region A is smaller than that of the second region B.

For example, it is preferable that the width W_{A1} at the side of the insert end part in the first region A is 10% to 80% of the width W_{B1} at the side of a removal end part in the second region B.

Specifically, assuming that: the dimension in the longitudinal direction of the absorptive member 2 is on the order of 65 mm; the dimension in the lengthwise direction of the surface member 1 is on the order of 65 mm; and the dimension in the widthwise direction of the surface member 1 is on the order of 127 mm, it is desirable that: the width W_{A1} at the side of the insert end part in the first region A is 5 mm to 40 mm, and is further preferably 10 mm to 20 mm; and the width W_{B1} at the side of the removal end part in the second region B is 40 mm to 80 mm, and is further preferably 50 mm to 60 mm.

If the width W_{A1} at the side of the insert end part in the first region A is 5 mm or less, since an area and a volume at a tip end part of the absorptive member 2 coming into contact with menstrual blood are small, such absorptive member 2 is easily folded inside of a finished product at the time of compressing and molding the absorptive member, thus leading to impaired liquid draw-in property.

In addition, if the width W_{B1} at the side of the removal end part in the second region B is 40 mm or less, since a difference in fiber density from the first region A is small, a sufficient liquid diffusion effect cannot be attained.

In addition, as shown in Fig. 3, the dimension in the widthwise direction in the absorptive member 2 may be continuously long as it goes from the side of the first region A to the side of the second region B. In such a case, the shortest dimension in the widthwise direction in the absorptive member 2 is the dimension W_{A1} in the widthwise direction at an end part of the first region A, and the longest dimension in the widthwise direction in the absorptive member 2 is the dimension W_{B1} in the widthwise direction at an end part of the second region B.

In the absorber 10 for the tampon that is obtained by compressing and molding such laminate member 10A, the fiber density after compressing, of a region that corresponds to the first region A, becomes lower than the fiber density of a region that corresponds to the second region B.

Specifically, in the absorber 10 for the tampon, the fiber density of the region that corresponds to the first region A is 0.17 g/cm³ to 0.32 g/m³, and the fiber density of the region that corresponds to the second region B is 0.37 g/cm³ to 0.52 g/cm³.

In addition, a surface shape of the absorptive member 2 before compressing and molding and a planer shape of the surface member 1 are not analogously similar to each other. For example, a difference between the area of the surface member 1 in the first region A and the area of the absorptive member 2 is greater than a difference between the area of the surface member 1 in the second region B and the area of the absorptive member 2.

In addition, the area of the surface member 1 in the first region A and the second region B may be greater than that of the absorptive member 2 in the first region A and the second region B.

Usually, the surface member 1 has rigidity which is lower than that of the absorptive member 2, and if the surface member is inserted into the vaginal opening, the above surface member flexibly follows (loosens) the behavior of the absorptive member 2 having inflated after absorbing liquid; and however, since the surface member 1 is not analogously similar to the absorptive member 2 and is greater in area at the insert side, the surface member 1 at the side that has been first inserted is likely to quickly broaden in width in the vaginal opening in accordance with the shape of the vaginal opening. In this manner, a gap between the vagina and the absorber 10 for the tampon is hardly formed, thus lessening the leakage exerted by liquid migrating rearward (to the wiped side) along the gap.

The pull-out cord 3 is comprised of a single yarn made of rayon, cotton, polyethylene, or polypropylene, or alternatively, a composite yarn formed by twisting them. Further preferably, the pull-out cord 3 may be processed in a water repelling manner with paraffin or the like in order to prevent staining exerted by menstrual blood or bodily fluid.

Here, in a case where the dimension in the lengthwise direction of the absorber 10 for the tampon after compressing and molding is 30 mm to 60 mm, it is preferable that a length of the pull-out cord 3 is within the range of 150 mm to 250 mm. If the length of the cord is 150 mm or less, the pull-out cord 3 is too short to find when the absorber 10 for the tampon is removed, or alternatively, if the length of the cord is 250 mm or more, there is apprehension that the cord comes into contact with cloth or toilet seat and then is stained after the absorber 10 for the tampon has been removed.

With the absorber 10 for the tampon, according to the first embodiment of the present invention, the dimension in the widthwise direction of the absorptive member 2 before compressing and molding is reduced at the side to be first inserted into the vaginal opening (the first region A) and is increased at the side to be first removed from the vaginal opening (the second region B), whereby, without providing the step of jetting any liquid, the fiber density at the side to be first inserted into the vaginal opening (the region corresponding to the first region) is reduced and then the fiber density at the side to be first removed from the vaginal opening (the region that corresponds to the second region B) is increased, in the absorber 10 for the tampon after compressing and molding.

With the absorber 10 for the tampon, according to the first embodiment of the present invention, at the time of absorption of liquid in the absorber 10 for the tampon, the liquid migrates from a region in which the fiber density is low, to a region in which the fiber density is high; and therefore, a slope in fiber density exists, whereby an initial absorption velocity to increases in particular; the liquid to migrate to the entirety of the absorber 10 for the tampon speedily; and the absorber 10 for the tampon is allowed to inflate quickly.

With the absorber 10 for the tampon, according to the first embodiment of the present invention, tilting is applied from an end part at the side to be first inserted into the vaginal opening (the region that corresponds to the first region A) to an end part at the side to be first removed from the vaginal opening (the region that corresponds to the second region B), thus increasing a distance at which the liquid runs along the absorber 10 for the tampon as well. As a result, even in a case where a large amount of menstrual blood has been observed, the menstrual blood stays in the absorber 10 for the tampon for a longer period of time, thus causing leakage to be unlikely to occur.

With the absorber 10 for the tampon, according to the first embodiment of the present invention, in a case where the dimension in the widthwise direction of the surface member 1 is determined so as to correspond to the maximum dimension in the widthwise direction of the absorptive member 2 before compressing and molding, the dimension in the widthwise direction of the absorptive member 2 at the side to be first inserted into the vaginal opening is smaller than the dimension in the widthwise direction of the absorptive member 2 at the side to be first removed from the vaginal opening (the region that corresponds to the second region B); and therefore, the surface member 1 at the side to be first inserted into the vaginal opening (the region that corresponds to the first region A) has a region with its low rigidity and its high degree of freedom, which is made of the surface member itself. As a result, the absorber can be flexibly taken along the fine fold of tissue in the vaginal opening where the absorptive member 2 is disallowed to enter, and no gap is formed, thus causing leakage to be unlikely to occur.

### (Absorber for Tampon According to Second Embodiment of the Present Invention)

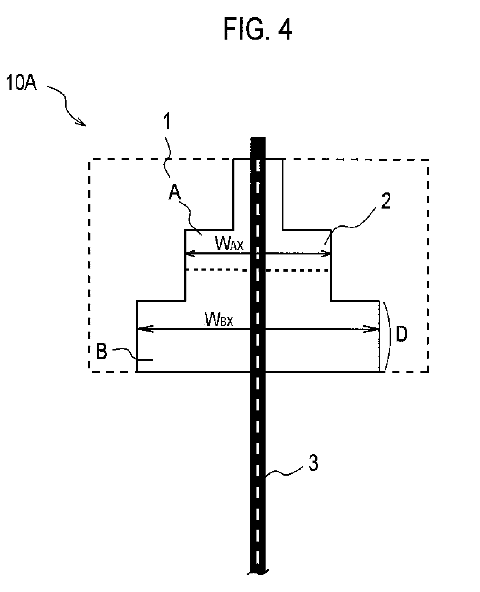

Hereinafter, with reference to Fig. 4, an absorber 10 for a tampon according to a second embodiment of the present invention will be described, focusing on differences from the absorber 10 for the tampon, according to the first embodiment mentioned above.

As shown in Fig. 4, in the illustrative embodiment, the dimension in the widthwise direction in the absorptive member 2 before compressing and molding is intermittently long, as it goes from the side of the first region A to the side of the second region B. Specifically, as shown in Fig. 4, a side face shape of the absorptive member 2 before compressing and molding is formed in a stepped shape.

The dimension in the widthwise direction in the absorptive member 2 before compressing and molding is constant at a predetermined distance D from an end part at the side of the second region B.

With the absorptive member for the tampon 10 according to the second embodiment of the present invention, a region in which the fiber density is low and a region in which the fiber density is high are formed before and after the stepped portion, whereby, in particular, the initial absorption velocity increases, thus allowing the absorber 10 for the tampon to initially inflate speedily.

Further, with the absorber 10 for the tampon, according to the second embodiment of the present invention, if liquid reaches up to a portion which broadens in width in a stepped shape, a region (an area) in which menstrual blood migrates in a widthwise direction increases, and a speed at which the menstrual blood migrates in a longitudinal direction becomes slow, accordingly; and therefore, it takes long until the liquid reaches up to an end part of the longitudinal direction of the second region, and the menstrual blood is more unlikely to leak.

Moreover, with the absorber 10 for the tampon, according to the second embodiment of the present invention, the absorptive member 2 exists in a crossing direction relative to a direction in which liquid flows; and therefore, the flow of the liquid is intercepted, and leakage is unlikely to occur at an end part at the side to be first wiped out from the vaginal opening (the region that corresponds to the second region B).

### (Absorber for Tampon According to Third Embodiment of the Present Invention)

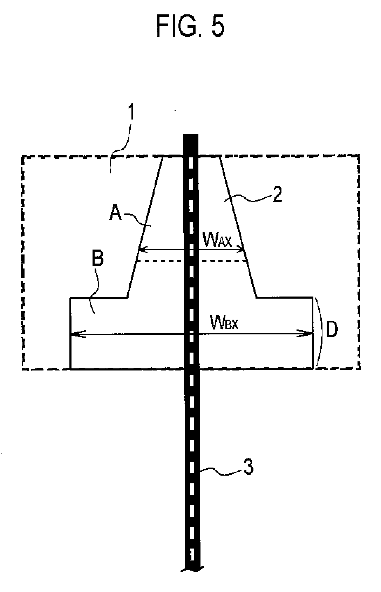

Hereinafter, with reference to Fig. 5, an absorber 10 for a tampon, according to a third embodiment of the present invention, will be described, focusing on differences from the absorber 10 for the tampon, according to the first and second embodiments mentioned above.

As shown in Fig. 5, in the illustrative embodiment, the dimension in the widthwise direction in the absorptive member 2 before compressing and molding is continuously long, as it goes from the side of the first region A to the side of the second region B, and in the second region B, the above dimension is intermittently long, as it goes from the side of the first region A to the side of the second region B. That is, specifically as shown in Fig. 5, in the second region B, a side face shape of the absorptive member 2 before compressing and molding is formed in a stepped shape.

The dimension in the widthwise direction in the absorptive member 2 before compressing and molding is constant at a predetermined distance D from an end part at the side of the second region B.

With the absorber 10 for the tampon, according to the third embodiment of the present invention, if liquid reaches up to the second region B, a region (an area) in which menstrual blood migrates in a widthwise direction increases, and a speed at which the menstrual blood migrates in a longitudinal direction becomes slow, accordingly; and therefore, it takes long until the liquid reaches up to an end part in the longitudinal direction of the second region B, and the menstrual blood is more unlikely to leak.

### (Absorber for Tampon According to Fourth Embodiment of the Present Invention)

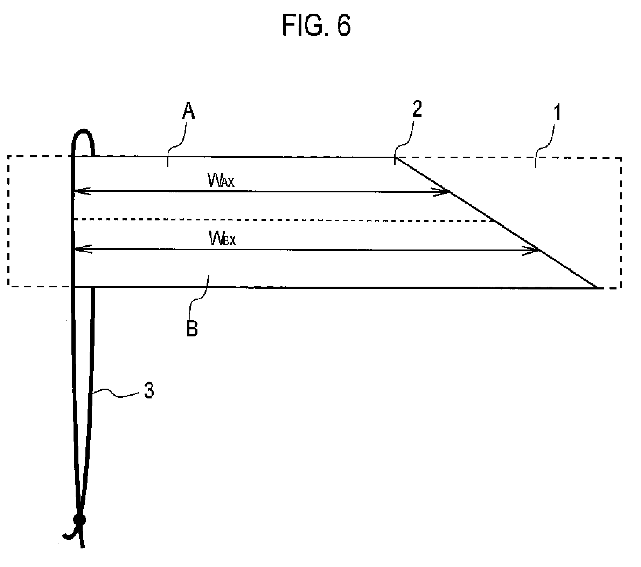

Hereinafter, with reference to Fig. 6, an absorber 10 for a tampon, according to a fourth embodiment of the present invention, will be described focusing on differences from the absorber 10 for the tampon, according to the first to third embodiments mentioned above.

As shown in Fig. 6, in the illustrative embodiment, an absorptive member 2 of roll type is structured so that: the member is folded into two sections after being formed in a trapezoidal shape; and further, the folded member is compressed and molded after being formed in a roll shape.

While the present invention has been thus described in detail by way of the foregoing embodiments, it is apparent to one skilled in the art that the present invention is not limitative to the embodiments described in the specification. The present invention can be carried out as modified and altered aspects without deviating from the gist and scope of the present invention defined by the claims. Therefore, the descriptive matters of the present specification are intended for the purpose of illustrative explanation, and do not have any limitative meaning to the present invention.

The entire contents of Japanese Patent Application No. 2008-281592 (filed on October 31, 2008) are incorporated in the present specification by reference.

### [Industrial Applicability]

As has been described above, an absorber for a tampon, according to the present invention is effective, since there can be provided an absorber for a tampon, which is capable of forming a region with its different fiber density from another one, without jetting any jetting any liquid to a laminate member.

## Claims

1. An absorber (10) for a tampon, which is generated by compressing and molding a laminate member (10A) including a liquid permeable surface member (1) and an absorptive member (2): wherein
the absorptive member before the compression and molding is comprised of: a first region (A) which is a region at a side to be first inserted into a vaginal opening in a case where the absorber for the tampon is divided into two sections in a longitudinal direction; and a second region (B) which is a region at a side to be first removed from the vaginal opening in a case where the absorber for the tampon is divided into two sections in a longitudinal direction; and
an area of the first region is smaller than an area of the second region;
wherein a width at a side of an insert end part in the first region is 10% to 80% of a width at a side of a removal end part in the second region;
wherein a surface shape of the absorptive member before the compression and molding and a planar shape of the surface member are not analogously similar to each other, and
a difference between an area of the surface member in the first region and an area of the absorptive member is greater than a difference between an area of the surface member in the second region and an area of the absorptive member.

2. The absorber for tampon according to claim 1,
a fiber density of a region which corresponds to the first region in the absorber for the tampon is 0.17 g/m³ to 0.32 g/cm³, and
a fiber density of a region which corresponds to the second region in the absorber for the tampon is 0.37 g/m³ to 0.52 g/cm³.

3. The absorber for the tampon according to claim 1,
a dimension in a widthwise direction in the absorptive member before the compression and molding is continuously or intermittently long, as the dimension goes from a side of the first region to a side of the second region.

4. The absorber for the tampon according to claim 1,
a dimension in a widthwise direction in the absorptive member before the compression and molding is constant at a predetermined distance from an end part at the side of the second region.

## Patentansprüche

1. Absorber (10) für einen Tampon, gebildet durch Kompression und Formen eines Laminatelements (10A) mit einem flüssigkeitsdurchlässigen Oberflächenelement (1) und einem saugfähigen Element (2), wobei
das saugfähige Element vor der Kompression und dem Formen umfasst: einen ersten Bereich (A), der auf einer Seite angeordnet ist, die in einem Fall, in dem der Absorber für den Tampon in Längsrichtung in zwei Abschnitte eingeteilt ist, zuerst in eine Vaginalöffnung eingeführt wird; und einen zweiten Bereich (B), der auf einer Seite angeordnet ist, die in einem Fall, in dem der Absorber für den Tampon in Längsrichtung in zwei Abschnitte eingeteilt ist, zuerst aus der Vaginalöffnung gezogen wird; und
wobei eine Oberfläche des ersten Bereichs kleiner ist als eine Oberfläche des zweiten Bereichs;
wobei eine Breite auf einer Seite eines Einführungsendes im ersten Bereich 10% bis 80% einer Breite auf einer Seite des Ausziehendes im zweiten Bereich beträgt;
wobei eine Oberflächenform des saugfähigen Elements vor der Kompression und dem Formen und eine ebene Form des Oberflächenelements einander nicht analog gleichen, und
ein Unterschied zwischen einer Fläche des Oberflächenelements im ersten Bereich und einer Fläche des saugfähigen Elements größer ist als ein Unterschied zwischen einer Fläche des Oberflächenelements im zweiten Bereich und einer Fläche des saugfähigen Elements.

2. Absorber für einen Tampon nach Anspruch 1, wobei
eine Faserdichte eines Bereichs, der dem ersten Bereich im Absorber für den Tampon entspricht, 0,17 g/m³ bis 0,32 g/m³ beträgt, und
eine Faserdichte eines Bereichs, der dem zweiten Bereich im Absorber für den Tampon entspricht, 0,37 g/m³ bis 0,52 g/m³ beträgt.

3. Absorber für einen Tampon nach Anspruch 1, wobei
eine Abmessung in einer Breitenrichtung im saugfähigen Element vor der Kompression und dem Formen kontinuierlich oder intermittierend lang ist, während die Abmessung von einer Seite des ersten Bereichs zu einer Seite des zweiten Bereichs geht.

4. Absorber für einen Tampon nach Anspruch 1, wobei
eine Abmessung in einer Breitenrichtung im saugfähigen Element vor der Kompression und dem Formen in einer vorgegebenen Entfernung von einem Endteil auf der Seite des zweiten Bereichs konstant ist.

## Revendications

1. Élément absorbant (10) pour tampon, qui est généré par la compression et le moulage d'un élément stratifié (10A) comprenant un élément de surface perméable aux liquides (1) et un élément absorbant (2) ; dans lequel
l'élément absorbant, avant la compression et le moulage, est constitué d'une première région (A) qui est une région au niveau du côté destiné à être inséré en premier dans une ouverture vaginale dans un cas de figure où l'élément absorbant du tampon est divisé en deux sections dans une direction allant dans le sens longitudinal ; et une deuxième région (B) qui est une région au niveau du côté destiné à être retiré en premier en provenance de l'ouverture vaginale dans un cas de figure où l'élément absorbant du tampon est divisé en deux sections dans une direction allant dans le sens longitudinal ; et
une zone de la première région est plus petite par rapport à une zone de la deuxième région ;
dans lequel une largeur au niveau du côté d'une partie d'extrémité d'insertion dans la première région représente de 10 % à 80 % d'une largeur au niveau du côté d'une partie d'extrémité de retrait dans la deuxième région ;
dans lequel une forme de surface de l'élément absorbant, avant la compression et le moulage, et une forme plane de l'élément de surface ne sont pas similaires de manière analogue l'une par rapport à l'autre, et
une différence entre une zone de l'élément de surface dans la première région et une zone de l'élément absorbant est supérieure par rapport à une différence entre une zone de l'élément de surface dans la deuxième région et une zone de l'élément absorbant.

2. Élément absorbant pour tampon selon la revendication 1,
une densité de fibres d'une région qui correspond à la première région dans l'élément absorbant du tampon va de 0,17 g/m³ à 0,32 g/cm³, et
une densité de fibres d'une région qui correspond à la deuxième région dans l'élément absorbant du tampon va de 0,37 g/m³ à 0,52 g/cm³.

3. Élément absorbant pour tampon selon la revendication 1,
une dimension dans une direction allant dans le sens de la largeur dans l'élément absorbant, avant la compression et le moulage, est longue de manière continue ou intermittente, la dimension allant d'un côté de la première région à un côté de la deuxième région.

4. Élément absorbant pour tampon selon la revendication 1,
une dimension dans une direction allant dans le sens de la largeur dans l'élément absorbant, avant la compression et le moulage, est constante à une distance prédéterminée depuis une partie d'extrémité du côté de la deuxième région.
